(19) Europäisches Patentamt
European Patent Office
Office européen des brevets

(11) EP 2 016 894 A1

(12) EUROPEAN PATENT APPLICATION

(43) Date of publication:
21.01.2009 Bulletin 2009/04

(51) Int Cl.:
A61B 5/0444 (2006.01)    G06F 17/00 (2006.01)

(21) Application number: 07112842.5

(22) Date of filing: 20.07.2007

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK RS

(71) Applicants:
• Stichting voor de Technische Wetenschappen
3527 JP  Utrecht (NL)
• Technische Universiteit Eindhoven
5612 AZ Eindhoven (NL)

(72) Inventors:
• Vullings, Rik
5801 AW, Venray (NL)
• Peters, Christiaan
5233 VA, Den Bosch (NL)

(74) Representative: DeltaPatents B.V.
Fellenoord 370
5611 ZL Eindhoven (NL)

(54) ECG signal processing

(57)    A system extracts an ECG signal from a composite signal (308) representing an electric measurement of a living subject. Identification means (304) identify a plurality of temporal segments (309) of the composite signal corresponding to a plurality of predetermined segments (202,204,206) of an ECG complex (208). A template generator (306) generates a plurality of respective template segments (310) corresponding to the respective predetermined segments (202,204,206), wherein a respective template segment is based on a plurality of the corresponding identified temporal segments, the template segments representing extracted portions of the ECG signal. A signal generator (312) combines a plurality of the template segments (310) to obtain an extracted ECG signal (314). The composite signal may comprise a maternal ECG signal and a fetal ECG signal, or the ECG signal and an EMG signal.

Fig. 4

EP 2 016 894 A1

**Description**

**Field of the invention**

The invention relates to ECG signal processing, and more particularly to extracting an ECG signal from a composite signal.

**Background of the invention**

Monitoring the fetal heart rate (fHR) and fetal electrocardiogram (fECG) in antenatal abdominal recordings is important to assess the fetal condition. The fHR variability provides physicians with information on the ability of the fetus to adapt to temporal oxygen deficiency and increments in the intra-uterine pressure. Moreover, the temporal and morphological parameters of the fECG provide additional information on fetal growth and its physiological condition.

The most widespread method to monitor the fHR is by means of Doppler ultrasound. This method is, however, associated with inaccuracies due to the fact that the ultrasound probe requires frequent repositioning because of fetal movement. In addition, the information provided by the fHR has a relatively low positive predictive value: it is only definite when the fetal condition is clearly good or clearly bad. For the situations in-between, the fECG could provide additional information to assess the fetal condition.

During labor, the fECG can be recorded by means of an electrode positioned directly on the fetus. This method is invasive and can only be applied when the fetal membranes have ruptured. Antepartum, the fECG can be monitored using contact electrodes positioned on the maternal abdomen. The signals recorded by these electrodes, however, are a mixture of electrophysiological signals, including the fECG, the maternal electrocardiogram (mECG), powerline interference from the electricity grid, and motion artifacts.

Since the spectral properties of the powerline interference and the motion artifacts differ from the spectral properties of the mECG and fECG, these interferences can be suppressed by frequency selective filtering. In contrast, the spectral properties of the fECG and mECG overlap and as a result these signals cannot be separated by filtering.

Several techniques to remove the mECG from antenatal abdominal recordings have been proposed in literature. One of the proposed classes of techniques, is the class of techniques that are based on template subtraction. In general, template subtraction techniques operate by generating a template of the mECG and subtracting this template from each individual mECG complex. One of the techniques in this class is described in "Basic aspects concerning the event-synchronous interference canceller" by M. Ungureanu et al., IEEE Transactions on Biomedical Engineering, Vol. 53, No. 11, November 2006. It describes an adaptive noise canceller (ANC), which is a linear system method for noise reduction in cases where the disturbing noise can be separately recorded as a reference signal and is not correlated with the signal of interest. An ANC modification has been proposed that uses an artificial reference signal based on event triggered averaging of segments of the recorded wanted (but disturbed) signal in order to obtain a template for the repetitive distortion sequence and to construct the artificial reference signal.

**Summary of the invention**

It is an object of the invention to provide an improved system for extracting an ECG signal from a composite signal. In a first aspect of the invention a system is presented that comprises

an input for receiving a composite signal representing an electric measurement of a living subject;
identification means for identifying a plurality of temporal segments of the composite signal corresponding to a plurality of predetermined segments of an ECG complex; and
a template generator for generating a plurality of respective template segments corresponding to the respective predetermined segments, wherein a respective template segment is based on a plurality of the corresponding identified temporal segments, the template segments representing extracted portions of the ECG signal.

The template segments provide a relatively accurate representation of segments of the ECG signal, because noise disturbances have been removed by considering a plurality of the identified temporal segments corresponding to a predetermined segment. Generating the templates for individual segments of the ECG complex provides better results than generating a large template (e.g. a template for a complete ECG complex) followed by an optional scaling of segments of the large template. This aspect of the invention allows to reduce inaccuracies that may be caused by a non-periodic behavior of the ECG signal. The template segments may for example be displayed to a user or used as an intermediate result subject to further processing. Preferably the plurality of the corresponding identified temporal segments are averaged. The temporal segments are preferably sequential temporal segments of the composite signal. Although the temporal segments may partially overlap, a temporal segment preferably contains a unique portion of the composite signal not covered by the other temporal segments. Similarly the predetermined segments of the ECG complex are preferably sequential predetermined segments of the ECG complex. A predetermined segment of the ECG complex preferably contains a unique portion of the ECG complex not covered by the other predetermined segments. The living subject may comprise a human or animal body. Preferably, a temporal segment corresponds to at least one of the predetermined segments, and one of the predetermined segments corresponds to more than one of the temporal segments.

An embodiment comprises a signal generator for combining a plurality of the template segments to obtain an extracted ECG signal. This results in a 'purified' ECG signal, i.e., an ECG signal from which the influence of disturbances caused by for example other signals and/or noise has been reduced.

An embodiment comprises a subtractor for subtracting the extracted ECG signal from the composite signal to obtain a residual signal. The residual signal may comprise valuable information, such as for example a fetal ECG signal or an EMG signal.

In an embodiment, the template generator comprises means for updating a template segment to take into account temporal segments in a predetermined time interval around or near a temporal segment of interest to obtain a time-varying adaptive template segment. This allows to keep the template segments up-to-date to changing conditions. For example, the amplitude or shape of a segment may vary over time, and this is taken into account by regularly updating the segment templates.

In an embodiment, means for subtracting the template segments from the corresponding temporal segments of the composite signal to obtain a residual signal. This allows to obtain a representation of the signals other than the ECG signal that are present in the composite signal. The template segments may first be combined into an extracted ECG signal before subtraction, however this is not required.

In an embodiment,

the input is arranged for receiving a maternal ECG signal and a fetal ECG signal mixed in the composite signal; and
the predetermined segments of the ECG complex are predetermined segments of a maternal ECG complex.

In this case, the residual signal provides a relatively high quality fetal ECG signal.

An embodiment comprises

a postprocessor for analyzing the residual signal, which comprises the fetal ECG signal; and
means for generating an alarm upon detecting a predetermined event in the fetal ECG signal.

This allows to provide a more automated monitoring system including an alarm that is triggered upon a clinical event.

In an embodiment, the input is arranged for receiving the ECG signal and an EMG signal mixed in the composite signal. The residual signal may then comprise a high quality EMG signal. EMG signals may also provide valuable clinical information.

In an embodiment, the predetermined segments of the ECG complex comprise at least part of at least one of: a P wave, a Q wave, an R wave, an S wave, a T wave. These waves form distinctly recognizable segments of the ECG complex, that are relatively easy to detect. Also, they may be conveniently scaled, time shifted and offset to match corresponding segments because each of these segments involves a single wave. To reduce the number of segments, it is possible to combine several waves in a single segment, for example either Q and R waves or R and S waves may be combined in a single segment or the QRS complex may be combined in a single segment.

In an embodiment, the identification means comprises a means for finding an extreme value and an adaptive threshold to identify a boundary of at least one of the segments. An adaptive threshold is an effective way to establish the bounds of a segment, because it may take into account any offset that may be present in the signal as well as the extreme value.

In an embodiment, the template generator comprises an outlier detector for detecting outliers among samples within a segment; and the template generator is arranged for not taking into account the outliers in determining the template segments. Outliers may be detected using for example a squared error criterion. These outliers deteriorate the quality of the template segment and accordingly they are preferably not taken into account in the computation of the template segment. However the non-outlier samples of the segment preferably are taken into account in the computation of the template segment.

An embodiment comprises a confidence estimator for providing a confidence measure of the composite signal based on the outliers to assess a relevance of an electrode used to acquire the composite signal in a multi-electrode configuration. The number of outliers or the severity of the outliers provides an indication of the suitability of the composite signal as a whole. Especially in a multi-electrode configuration this information may be used to compare the quality of the different signals acquired with the different electrodes.

In an embodiment, the template generator comprises a data fitter for fitting a temporal segment to a template segment by at least one of: scaling, applying a time shift, or applying an offset. Applying a scale factor, a time shift, and/or an offset is an efficient way to prepare the segments for averaging and/or for detecting outliers with a high level of robustness. Preferably all three are applied.

In an embodiment, a capacitive electrode is used for acquiring the composite signal. Capacitive electrodes are relatively comfortable because they do not need to be fixed onto the skin by using adhesive.

An embodiment comprises at least one of:

a display for showing at least one of the established signals;
a recorder for storing at least one of the established signals; or
an output for transmitting at least one of the established signals to another device.

This enhances the usability of the system in a clinical setting. This usability may be further enhanced by implementing the system in a cardiac monitoring device.

An embodiment comprises a method of extracting an ECG signal from a composite signal, the method comprising

receiving a composite signal representing an electric measurement of a living subject;
identifying a plurality of temporal segments of the composite signal corresponding to a plurality of predetermined segments of an ECG complex; and
generating a plurality of respective template segments corresponding to the respective predetermined segments, wherein a respective template segment is based on a plurality of the corresponding identified temporal segments, the template segments representing extracted portions of the ECG signal.

An embodiment comprises a computer program product comprising machine executable instructions for causing a processor to perform the method set forth.

**Brief description of the drawings**

These and other aspects of the invention will be further elucidated and described with reference to the drawing, in which

Fig. 1A illustrates an embodiment;
Fig. 1B illustrates an embodiment;
Fig. 2 illustrates an ECG signal;
Fig. 3 illustrates a T wave;
Fig. 4 illustrates an embodiment; and
Fig. 5 illustrates an embodiment.

**Detailed description of embodiments**

Monitoring the fetal heart rate (fHR) and fetal electrocardiogram (fECG) during pregnancy is important to support medical decision-making. Before labor, the fHR is usually monitored using Doppler ultrasound. This method is inaccurate and has a low positive predictive value. During labor, the fHR can be monitored more accurately using an invasive electrode; this method also enables monitoring of the fECG. Antenatally, the fECG and fHR can also be monitored using electrodes on the maternal abdomen. The signal to noise ratio of these recordings is, however, low, the maternal electrocardiogram (mECG) being the main interference. Existing techniques to remove the mECG from these non-invasive recordings are insufficiently accurate or do not provide all spatial information of the fECG. In this text a technique for mECG removal in antenatal abdominal recordings is presented. The technique operates by the linear time-invariant prediction of each separate wave in the mECG. The technique may outperform spatial filtering, adaptive filtering, and template subtraction in mECG removal and may perform better than ICA in fHR detection.

Monitoring the fetal electrocardiogram (fECG) and heart rate variability is currently considered one of the most promising methods to assess fetal health. Fetal heart rate (fHR) variability is associated with stimulation of the autonomous nervous system and consequently provides information about the development of this system. Moreover, during uterine contractions, fHR variability provides information about the capability of the fetus to adapt to alternative circumstances, i.e. temporary reduction in the oxygen supply and increased pressure to the fetal head. The temporal and morphological properties of the fECG provide information about fetal hypoxemia and condition.

During labor, the fECG can be monitored using an electrode connected directly to the fetus. In recent years, an extensive fECG analysis technique has been developed to support physicians in diagnosing fetal oxygen deficiency. However, the required direct electrode can only be applied when the fetal membranes have ruptured and entails an increased risk of infection due to its invasiveness.

In stages of pregnancy earlier than labor, Doppler ultrasound is the most widely used method to monitor the fHR. This method is, however, inaccurate due to the the low signal to noise ratio of the Doppler signals and exhibits a relatively low positive predictive value: it can only determine when the fetal condition is clearly good or clearly bad. Reasons for the low signal to noise ratio of the Doppler signals are the small dimensions of the fetal heart and vessels and the fact that the ultrasound probe needs frequent repositioning because of movement of the fetus. Moreover, information of the fECG is not available when using Doppler ultrasound.

Antepartum, the fHR and fECG can be monitored with abdominal contact electrodes. The signals recorded by these electrodes are a mixture of electrophysiological signals and noise and therefore require signal processing to extract the fECG and fHR. Some of the predominant interferences are the maternal electrocardiogram (mECG), the powerline interference from the electricity grid, and motion artifacts. Since the powerline interference and motion artifacts have different spectral properties from the mECG and fECG, these interferences can be suppressed by frequency selective filtering. The spectra of the mECG and fECG, however, overlap and as a consequence cannot be separated by means of filtering.

Several techniques to remove the mECG from abdominal recordings have been proposed in literature. The majority of these techniques is based on spatial filtering, adaptive filtering, and template subtraction. Spatial and adaptive filtering assume a certain degree of correlation between the mECG recorded at different locations, while template subtraction techniques assume a (quasi-)stationary behavior of the mECG. The main problems associated with these techniques are in these assumptions. To fully exploit the correlation between the mECG signals at different locations, the mECG reference signals should be uncorrupted or strictly positioned on the maternal abdomen. However, because it is not possible to record the mECG reference signals without noise or at exact positions, mECG removal by means of spatial and adaptive filtering is suboptimal. Template subtraction techniques do not operate optimally because consecutive mECG complexes normally vary not only in amplitude but, as a result of respiratory effects, also in morphology. This non-stationary behavior causes inaccurate removal of the mECG.

In recent years, the use of blind source separation (BSS) techniques for fECG extraction has steadily gained interest. The most prominent BSS technique that has been employed is independent component analysis (ICA) which is based on the assumption that the recorded signals are composed of a mixture of signals from several statistically independent sources. Similar to template subtraction techniques, ICA also assumes some degree of stationarity in the recorded signals in order to determine the mixing of the independent sources.

In this text a technique is presented for the removal of the mECG. To some extent, this technique overcomes the problems concerning strict electrode placement and non-stationary behavior of the signals that are associated with previously described mECG removal techniques. This technique is an extension of mECG template subtraction techniques and is referred to as the weighted averaging of mECG segments (WAMES). WAMES operates by dividing the mECG complex into separate segments and dynamically generating an estimate for each individual segment. Each estimate is hereby obtained by the linear combination of offset compensated, scaled, and time-shifted corresponding segments in preceding mECG complexes. This process can also be referred to as the dynamic segmentation and linear prediction of mECG segments. By considering segments individually, non-stationarities in the mECG can be dealt with accurately while the quasi-stationarity of the mECG is still exploited, viz. the scaling and offset compensation of the mECG segments enables WAMES to deal with non-stationary changes in the morphology of the mECG, whereas the generation of an estimate for each individual segment by the linear combination of corresponding segments in preceding mECG complexes is based on the assumption that the mECG is a quasi-stationary signal. As stated previously, some of the main interests in removing the mECG from antenatal abdominal recordings are to obtain the fHR and fECG.

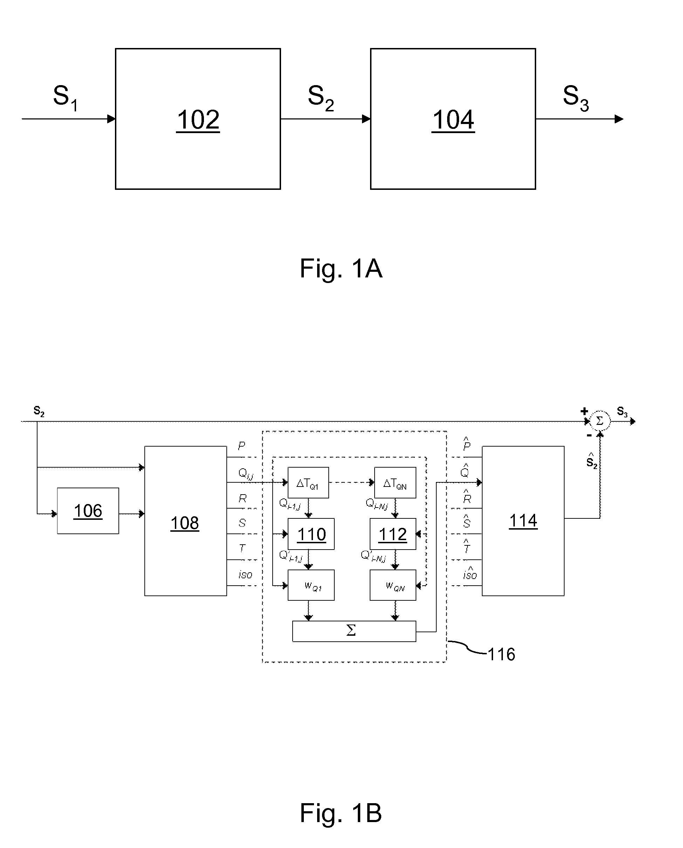

Antenatal abdominal recordings generally constitute a mixture of fECG, mECG, and noise such as motion artifacts, muscular activity, and powerline interference. Consequently, most fECG extraction techniques operate by means of consecutive removal of each interference. Schematically this can be seen as the two step procedure in Fig. 1A. Removal of motion artifacts, muscular activity, and powerline interference is referred to as preprocessing block 102 in Fig. 1A and is not discussed in detail in this text. Methods are known in the art to address these issues. The abdominal recordings are represented in Fig. 1A and Fig. 1B by $S_1$, which is a $[n \times m]$ matrix with $n$ the number of electrodes positioned on the maternal abdomen and $m$ the number of samples in the recording. The preprocessed signals $S_2$, with $S_2$ also a $[n \times m]$ matrix, are subsequently fed into the mECG cancellation block 104; for WAMES this block is shown schematically in Fig. 1B.

Fig. 1B shows a block scheme of the mECG cancellation by WAMES. The pre-processed signal $S_2$ is provided to a maternal QRS detection block 106. Maternal QRS complexes may be detected because of their large amplitude. The pre-processed signal $S_2$ and the detected QRS complexes are provided to a dynamic mECG segmentation block 108. This dynamic mECG segmentation block 108 divides the signal into a plurality of temporal segments, wherein each segment corresponds to one of the characteristic portions of the ECG complex: P, Q, R, S, T wave and isoelectric periods. The mECG segmentation block 108 first does an initial guess of a time interval in which the different segments may be found based on the location of the QRS complexes. Then, the actual position of the wave is determined based on a maximum value within the guessed time interval and an adaptive threshold is used to set the boundaries of the segment. The segments thus established are provided to linear prediction block 116. In Fig. 1B, the linear prediction block 116 is illustrated for a Q wave; however, a similar structure applies to the processing of the other types of segments. The delays $\Delta T_{Q_1} ... \Delta T_{QN}$ correspond to the time intervals between the Q-wave that is estimated and $N$ preceding Q-waves. These time intervals are approximated in the maternal QRS detection block 106, in which the time intervals between consecutive R-waves are determined. Align and scale blocks 110 and 112 perform the fitting of the individual segments, as will be elucidated hereinafter. The weights $w_{Q1}, ..., w_{QN}$ represent the weights associated with the time intervals between the Q-wave that is estimated and $N$ preceding Q-waves. The values of these weights will be explained hereinafter. The summation block $\Sigma$ performs the weighted averaging of the aligned and scaled waved of preceding mECG complexes. As noted before, similar processing is performed for the other waves (P, R, S, T, iso). The resulting weighted averaged segments are provided to mECG segment combination block 114. Here, the individual segments are combined into a continuous signal representing the maternal ECG. This continuous signal is subtracted from the original pre-processed signal $S_2$ to obtain the signal $S_3$. The signal $S_3$ contains all the signals present in $S_2$, except for the maternal ECG signal which has been removed.

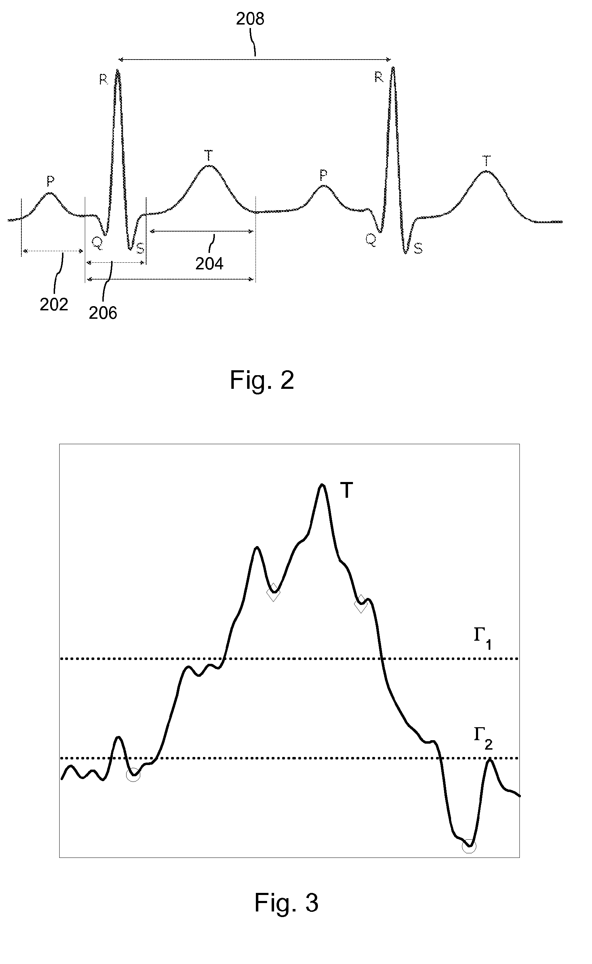

Fig. 2 illustrates an ECG complex and nomenclature associated therewith. ECG complexes often consist of a P-wave, associated with the depolarization of the atria, a QRS-complex, which can be subdivided into a separate Q-wave, R-wave, and S-wave and associated with the depolarization of the ventricles, and a T-wave that is associated with the repolarization of the ventricles. Each mECG complex in the preprocessed signals $S_2$, used as input for the mECG cancellation block 104 of Fig. 1A, is segmented into individual waves, i.e. a separate P-, Q-, R-, S-, and T-wave. The boundaries of these segments (or waves) are determined with respect to the position of the maternal QRS-complexes. Each segment is subsequently estimated by linear time-invariant prediction, using corresponding segments from preceding mECG complexes. This prediction consists of aligning and scaling preceding segments to increase the rate of agreement with the segment that is estimated, and the weighted averaging of the aligned and scaled preceding segments. These estimated segments are finally combined to generate an estimate $\hat{S}_2$ of the mECG signal.

Depending on the position of the electrodes on the maternal abdomen with respect to the position of the heart, some of the waves in Fig. 2 can be difficult to distinguish. Therefore, to facilitate the detection of maternal QRS-complexes in the maternal QRS detection block 106, the signal to noise ratio of the maternal QRS-complexes is enhanced by linearly combining the signals $S_2$ in such way as to maximize the variance (principal component analysis, PCA, which is a known technique). The linear combination with maximum variance is referred to as the principal component $pc$.

Maternal QRS-complexes are detected in this principal component as local extrema that exceed an adaptive threshold $\mu$. This threshold is updated continuously by means of a Kalman filter and depends on the signal to noise ratio of the maternal QRS-complexes in the principal component; when the signal to noise ratio changes the threshold is adapted to prevent noise from exceeding it. This Kalman filter is defined as

$$\mu_t = \mu_{t-1} + K_t \left( \nu_t - \mu_{t-1} \right), \qquad (1)$$

with $\nu_t$ the instantaneous threshold value at time $t$ defined as

$$\nu_t = \xi_1 \max L_I + \xi_2 \min L_I, \qquad (2)$$

with $\xi_1$ and $\xi_2$ parameters that are preferably set at or near 0.6 and 0.4, respectively, based on the data, $I$ a time interval of 1 second preceding time $t$ and $L_t$ the absolute gradient of the principal component $pc$, averaged over a time interval $T$ of preferably at or around 50 ms preceding $t$, containing $N_T$ samples:

$$L_t = \frac{1}{N_T} \sum_{j=t-N_T}^{t} \left| pc_{j+1} - pc_j \right|. \qquad (3)$$

Here $K_t$ is the Kalman gain defined as

$$K_t = \frac{\rho_t^2}{\rho_t^2 + \sigma_{pc_I}^2}, \qquad (4)$$

with

$$\rho_t^2 = \frac{\rho_{t-1}^2 \sigma_{pc_I}^2}{\rho_{t-1}^2 + \sigma_{pc_I}^2} \qquad (5)$$

the variance of the estimate of $\mu_t$ at time $t$ and $\sigma_{p_I}^2$ the variance of $pc$ in the interval $I$.

At a certain distance the heart can be modeled by a time-dependent dipole with variable amplitude and orientation. In this framework, the ECG can be seen as the projection of the electrical field generated by this dipole on the measurement vector. Respiration causes motion of the maternal abdomen and as a result, the orientation of the measurement vector with respect to this electrical field varies over time. As each wave in the ECG exhibits its own orientation of the dipole, this variation is not proportional for each wave and therefore causes variability in the morphology of the ECG. This morphological variability is the main reason for inaccuracies in existing template subtraction techniques. The template is not capable of accounting for all variations and consequently residuals of the mECG remain after subtraction of the template. These residuals can have amplitudes that exceed the amplitude of the fECG and therefore affect detection of the fHR. By generating a template for each individual wave the morphological variability can be accounted for, resulting in an improved mECG subtraction.

Segmentation of the mECG complex into individual waves is performed in block 108 by means of windowing and adaptive thresholds. At first, for each wave a rectangular window is defined in which the start and end of the wave will be detected. Secondly, the actual detection of the start and end of the wave is performed by using an adaptive threshold.

The temporal parameters of the individual waves in the ECG are related to conductive and geometrical properties of the heart. These temporal parameters constitute the moment of incidence and the width of the waves and therefore define the windows in which the waves can be detected. The moments of incidence for each wave are hereby defined with respect to the peak in the QRS-complex.

As stated before, the P-wave is associated with depolarization of the atria. The width of the wave is therefore associated with the size of the atria and the conduction speed of the action potential through the atrial tissue. For simplicity, both the atrial size and conduction speed are assumed to be proportional to the size and conduction speed of the ventricles. The width of the window for detecting the P-wave is therefore set proportional to the width of the QRS-complex since this width is associated with the conduction of the action potential through the ventricles. The moment of incidence of the P-wave window is determined by the length of the P-R interval 202 (Fig. 2), which depends on the conduction speed through the AV node. This conduction speed is regulated by the autonomous nervous system. As this system is also responsible for heart rate variability, the moment of incidence of the P-wave window is set dependent on the maternal heart rate.

Definition of the window for detecting the T-wave is analogous to the definition of the P-wave window. The main difference is that the length of the R-T interval 204 is set at a fixed value since the length of this interval is not regulated by neural stimulation; the width of the T-wave window is therefore set proportional to the width of the QRS-complex, the QRS-interval 206. For the individual waves in the QRS-complex, i.e. the Q-wave, the R-wave, and the S-wave, both temporal parameters, i.e. the moment of incidence and the width of the wave, are set at a fixed value, assuming the absence of cardiac pathologies like bundle branch blocks. The fixed values for both the R-T interval 204 and the QRS-complex are chosen small enough to enable WAMES to deal with high maternal heart rates adequately.

The start and end of each wave may be defined in the windows as the first local extrema on either side of the peak of that particular wave. However, generally the P-wave and T-wave have a low signal to noise ratio and as a result the detection of the start and end of the wave is affected by noise. By defining an adaptive threshold that depends on the signal to noise ratio within the window and performing the detection of the local extrema for the samples that do not exceed this threshold, this problem can be overcome. This threshold is determined in two steps. At first, the mean value $\Gamma_1$ of the samples within the window is determined. Secondly, the mean value $\Gamma_2$ of the samples with absolute value smaller than $\Gamma_1$ is determined and used as threshold. Advantage of this method is that high noise levels within the window may cause an increase in the threshold value whereas low noise levels result in a lower threshold value.

Fig. 3 shows an example of the detection of the start and end of a T-wave. The threshold $\Gamma_2$ is indicated with the lower dotted line. The symbols '◊' indicate the detected positions of the start and end of the depicted T-wave when the threshold is not used; the '○' indicate the detected positions of the start and end when the threshold is employed.

Summarizing, the moment of incidence of the P-wave is defined as variable, whereas the moments of incidence of the Q-wave, R-wave, S-wave, and T-wave are fixed. In addition, the widths of the P-wave and T-wave are defined as variable and the widths of the Q-wave, R-wave, and S-wave are fixed.

To improve robustness of the segmentation, the quasi-stationarity of the mECG may be exploited; waves in consecutive mECG complexes usually do not occur and are preferably not detected at locations that differ too much from each other, e.g. more than 10% variation in the detected locations between consecutive mECG complexes is unlikely. This restriction reduces the misdetection of mECG waves due to signal distortion by fECG complexes. When, for example, the start of a particular wave is detected incorrectly due to the presence of a fECG complex, this restriction causes the misdetected start to be adjusted towards a value corresponding to the start of the similar wave in the preceding mECG complex. Waves that show too much variability in width and the locations of the start and end are assumed to be difficult to distinguish and they are therefore included in the isoelectrical periods. These isoelectrical periods are the parts of the signal that are not included in the individual waves. These periods are associated with zero amplitude of the dipole or perpendicular orientation of the measurement vector with respect to the electrical field.

In the linear prediction block 116, each wave in the mECG is estimated by the weighted averaging of *N* corresponding waves in preceding mECG complexes. Prior to averaging, the waves are aligned by synchronization for example on the start of the wave. As a result of the improved robustness in the mECG segmentation, however, the accuracy of the segmentation may be reduced and consequently this alignment can be inaccurate up to a few sampling periods. To improve the alignment the waves are synchronized by minimizing the mean squared error (MSE):

$$MSE\left(\theta_{i-k}\right) = \frac{1}{M}\sum_{j=1}^{M}\left(Z_{i,j} - Z_{i-k,j+\theta_k}\right)^2, \theta_k \in Z, \tag{6}$$

with *M* the length of the wave, $\theta_{i-k}$ the integer shift, $Z_{i,j}$ the wave that is estimated and $Z_{i-k,j}$ the corresponding wave of the $k^{th}$ preceding mECG complex ($Z = \{P,Q,R,S,T,iso\}$, *iso* =isoelectrical period). Each of the preceding waves $Z_{i-k,j}$ is thus shifted over a length $\theta_k$ that corresponds to the minimum MSE for that particular wave.

Due to respiration the DC component and amplitude of the preceding mECG waves $Z_{i-k,j}$ differ from the DC component and amplitude of the current mECG wave $Z_{i,j}$. Moreover, because of the finite sampling frequency (for example 1 kHz), misalignments smaller than one sampling period are expected, which may limit the accuracy of the estimation. To improve accuracy, the DC component and amplitude of $Z_{i-k,j}$ are preferably scaled properly and the time-shift has to be extended by shifts smaller than one sampling period. To calculate the optimal parameters, in a least mean squared (LMS) error sense, $Z_{i-k,j}$ requires interpolation to obtain quasi-continuous signals. Hence interpolation is applied on each sample. For example a parabolic interpolation scheme is used. For each sample a parabola is fitted through that particular sample and the adjacent samples on either side:

$$\tilde{Z}_{i-k,j} = \alpha_j j^2 + \beta_j j + \gamma_j. \tag{7}$$

Here $\tilde{Z}_{i-k,j}$ is the parabolically interpolated wave and $\alpha_j$, $\beta_j$, and $\gamma_j$ are the parabolic coefficients. For consecutive samples *j* these coefficients can be calculated by:

$$\alpha_j \;\; = \;\; \frac{1}{2}Z_{i-k,j-1} - Z_{i-k,j} + \frac{1}{2}Z_{i-k,j+1}$$

$$\beta_j \;\; = \;\; -\left(j+\frac{1}{2}\right)Z_{i-k,j-1} + 2jZ_{i-k,j} - \ldots$$

$$\ldots -\left(j-\frac{1}{2}\right)Z_{i-k,j+1} \tag{8}$$

$$\gamma_j \;\; = \;\; \frac{1}{2}j(j+1)Z_{i-k,j-1} - \ldots$$

$$\ldots -\left(j^2-1\right)Z_{i-k,j} + \frac{1}{2}j(j-1)Z_{i-k,j+1}.$$

The reason for using parabolic interpolation is that it is the lowest-order polynomial interpolation that can account for local extrema and has a continuous first derivative. Moreover, its computational simplicity is favored over more complex interpolation schemes. However other known interpolation techniques may be applied instead. The DC offset compensated, scaled, and time-shifted wave $Z'_{i-k,j}$ can be calculated by:

$$Z'_{i-k,j} = a\tilde{Z}_{i-k,j+b} + c, \tag{9}$$

$$a \in R, b \in \{R \cap (-\Delta t_s, \Delta t_s)\}, c \in R, \tag{10}$$

with $a$ the scaling parameter, $b$ the required time-shift, $c$ the DC component, and $\Delta t_s$ the length of one sampling period. The optimal parameters $\hat{a}$, $\hat{b}$, and $\hat{c}$ are calculated by minimizing the MSE between the wave that is estimated $Z_{i,j}$ and the offset compensated, scaled and time-shifted wave $Z'_{i-k,j}$:

$$\vec{\nabla}\left(\frac{1}{M'}\sum_{j \in F_{i-k}}\left(Z_{i,j} - Z'_{i-k,j}\right)^2\right) = \vec{0}, \tag{11}$$

where $\vec{\nabla}$ stands for the gradient $\left(\frac{\partial}{\partial a}, \frac{\partial}{\partial b}, \frac{\partial}{\partial c}\right)$. Here $F_{i-k}$ is the set of samples that do not contain artifacts or fECG complexes and $M'$ is the number of samples included in $F_{i-k}$.

Artifacts and fECG complexes may affect the calculation of the parameters $\hat{a}$, $\hat{b}$, and $\hat{c}$ and therefore reduce the accuracy of mECG estimation. By excluding samples that possibly contain artifacts and fECG complexes from the calculation of these parameters, this accuracy can be improved. Artifacts and fECG complexes generally distort the mECG wave. Consequently, comparison between waves with normalized amplitudes provides information about the rate of distortion. Moreover, as in general artifacts and fECG complexes extend over some time, the use of a sliding window benefits the detection of these artifacts or fECG complexes. Samples containing artifacts or fECG complexes can be detected by determining the MSE between two normalized waves within a sliding window. When the windowed MSE exhibits a distinct localized maximum, this maximum indicates a local distortion within one of the waves and thus a possible artifact or fECG complex.

The waves $Z_{i,j}$ in the mECG are estimated by the weighted averaging of the aligned and scaled waves of preceding mECG complexes $Z'_{i-k,j}$ (see Equation (10)). The weights used in this averaging are determined as the reciprocals of the MSE:

$$w_{i-k} = \left( \frac{1}{M'} \sum_{j \in F_{i-k}} \left( Z_{i,j} - Z'_{i-k,j} \right)^2 \right)^{-1}, \tag{12}$$

with $F_{i-k}$ the set of samples that do not contain artifacts or fECG complexes and $M'$ the number of samples included in $F_{i-k}$.

Since several preceding waves are included in the averaging, it can occur that both $Z_{i,j}$ and $Z_{i-k,j}$ for a particular value of $k$ are similarly distorted by a fECG complex. Consequently, exclusion of samples from $F_{i-k}$ can be erroneous and as a result the weight for averaging $w_{i-k}$ can be too large: a similar distortion of $Z_{i,j}$ and $Z_{i-k,j}$ causes the MSE to be relatively small and hence the weight to be relatively large. Furthermore, it can occur that preceding waves suffer from large distortions, resulting in relatively small weights $w_{i-k}$. These waves, however, still affect the averaged estimate of $Z_{i,j}$. To overcome these effects, only preceding waves are included in the weighted averaging for which the weights $w_{i-k}$ are in the interval:

$$\overline{w} - \sigma_w \leq w_{i-k} \leq \overline{w} + \sigma_w, \tag{13}$$

with $\overline{w}$ the mean weight and $\sigma_w$ the square root of the weight variance.

The average mECG wave $\hat{Z}_{i,j}$ can subsequently be calculated by:

$$\hat{Z}_{i,j} = \frac{\sum_{k \in G} w_{i-k} Z'_{i-k,j}}{\sum_{k \in G} w_{i-k}}. \tag{14}$$

Here $G$ is the set of preceding waves with weights satisfying Equation (13). The maximum number of waves in $G$ is restricted to the number of preceding waves $N$ included in the estimation of the mECG. Assuming that the fECG and mECG are not synchronized and disregarding the scaling of the waves, the amplitude of the fECG in the average mECG wave $\hat{Z}_{i,j}$ is reduced by a factor equal to the number of waves included in $G$. The desired reduction of the fECG amplitude in the averaging is empirically set at 10, however other values may be used. Since only preceding waves with weights $w_{i-k}$ satisfying Equation (13) are included in the averaging, the number of preceding waves $N$ included in the estimation has to exceed 10. Assuming a Gaussian distribution of the weights, Equation (13) results in $N = 15$.

In the mECG segment combination block 114, the individual average mECG waves $\hat{Z}_{i,j}$ are combined into an estimate of the mECG signal $\hat{S}_2$ (see Fig. 1A) by means of linear interpolation on the segment transitions.

An important advantage of the method described above is that scaling is performed on the individual mECG segments rather than on the template segments. Moreover, in WAMES each segment is individually time-aligned, resulting in a more accurate mECG estimate, in particular in the event of varying time delays between the individual ECG waves.

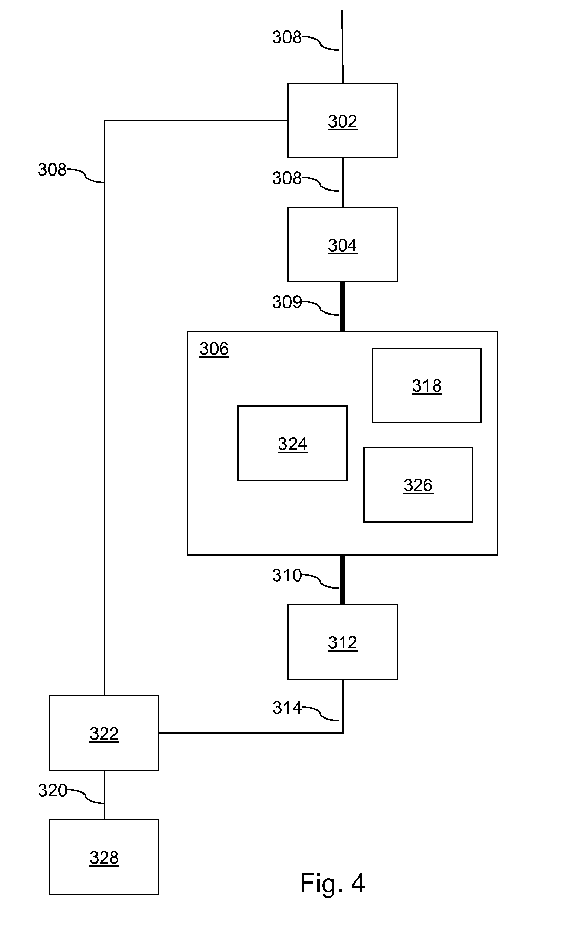

Fig. 4 illustrates an embodiment of the invention. The figure shows schematically a system for extracting an ECG signal from a composite signal 308. The composite signal 308 is received by input 302, for example by means of electrode measurement. The input 302 may receive preprocessed signal $S_2$, or it may comprise pre-processing capabilities to transform a raw signal $S_1$ into a preprocessed signal $S_2$ (not shown). Alternatively, the whole system may use the raw signal $S_1$ without preprocessing. The composite signal 308 is forwarded to an identification means 304, wherein a plurality of temporal segments 309 of the composite signal are identified. These temporal segments 309 correspond to a plurality of predetermined segments of an ECG complex 208. For example, the typical waves P, Q, R, S, T, and the isoelectrical periods are the predetermined segments (see Fig. 2). In this case, each temporal segment is an actually measured P, Q, R, S, or T wave or isoelectrical period. However, any division into predetermined segments is possible; for example, the ECG complex may be subdivided into a plurality of equal length segments. One or more of the P,Q,R, S,T waves may be subdivided into several predetermined segments, or a plurality of the waves may be combined in a single predetermined segment; for example, the Q, R, and S waves may be combined in a single predetermined segment.

A template generator 306 generates templates for each of the predetermined segments. Preferably the templates are adaptive, i.e., they adapt to any morphological or other changes in time as measured in the composite signal. However, the templates do not comprise any of the noise or any other signals apart from the ECG signal that is extracted. The template generator 306 generates a plurality of respective template segments 310 corresponding to the respective predetermined segments, wherein a respective template segment is based on a plurality of the corresponding identified temporal segments. For example, the plurality of the corresponding identified temporal segments are averaged.

A signal generator 312 is provided for combining the generated template segments 310 to obtain an extracted ECG signal 314. For example, the template segments are concatenated and/or interpolated, taking into account any time shifts/scaling/offset as measured in the actual signal. this results in a purified extracted ECG signal 314. It is purified because any other signals, including noise and/or signals from muscle activity and/or signals from a fetus, such as a fetal ECG, are removed from the extracted ECG signal 314.

A subtractor 322 is provided for subtracting the extracted ECG signal 314 from the composite signal 308 to obtain a residual signal 320. This residual signal 320 accordingly does not comprise the extracted ECG signal 314, but it does comprise the other signals of the composite signal, including noise and/or signals from muscle activity and/or signals from a fetus, such as a fetal ECG.

The template generator 306 comprises means 318 for updating a template segment. The means 318 takes into account temporal segments in a predetermined time interval around or near a temporal segment of interest to obtain a time-varying adaptive template segment. The predetermined time interval comprises for example the last $N$ ECG complexes, or $N/2$ ECG complexes before the segment of interest and $N/2$ ECG complexes after the segment of interest. Here, $N$ is a predetermined constant, for example 20.

In a preferred embodiment, the input is arranged for receiving a maternal ECG signal and a fetal ECG signal comprised in the composite signal. Accordingly, the predetermined segments of the ECG complex are predetermined segments of a maternal ECG complex. The residual signal comprises the fetal ECG signal. A postprocessor may be provided for analyzing the residual signal, which comprises the fetal ECG signal. For automatic monitoring of fetal condition, a means is provided for generating an alarm upon detecting a predetermined event in the fetal ECG signal.

Alternatively, the input is arranged for receiving the ECG signal and an EMG signal comprised in the composite signal. In this case, the residual signal comprises the EMG signal. It is also possible that the composite signal comprises maternal ECG signal, fetal ECG signal, and maternal or fetal EMG signal, and the residual signal comprises the fetal ECG signal and the EMG signal.

The identification means 304 comprises a means for finding an extreme value and an adaptive threshold to identify a boundary of at least one of the segments. As explained above, each of the P, Q, R, S, T waves is characterized by a curve having a maximum or a minimum (i.e., an extreme value) in the middle and descending towards the isoelectrical level both to the left and to the right of the extreme value. Accordingly, a bound of a segment may be found by noticing where the curve crosses an adaptive threshold, where the adaptive threshold is somewhere between the extreme value and the typical isoelectrical value.

The template generator 306 comprises an outlier detector 324 for detecting outliers among samples within a segment. For example, if a fetal R-wave occurs, this may cause such outliers. The template generator 306 is arranged for not taking into account the outliers in determining the template segments. However, the remaining, non-outlier portion of the segment is taken into account in determining the template segments.

The outlier detector 324 compares a temporal segment with a preliminary template segment. The temporal segment is subtracted from the preliminary segment, and the differences are used to find samples that are possibly corrupted by artifacts (e.g., fetal QRS complexes). These corrupted samples are excluded from the calculation of the alignment and from the scaling process. As the process is performed sequentially, for each of the preceding segments, samples are known that possibly contain artifacts (because this validation procedure was performed before on each of the preceding segments). These samples are therefore also excluded from the calculation of alignment and scaling parameters. The final template generated by the template generator 306 takes into account all the temporal segments, from which the corrupted samples have been removed.

A confidence estimator may be provided to establish a confidence measure of the composite signal based on the outliers. For example, the number of outliers per unit of time is determined by counting. This may be used to assess a relevance of an electrode used to acquire the composite signal in a multi-electrode configuration. In case of further processing of the signals, one may determine which measured signals to use for further processing based on the confidence measure.

The template generator 306 comprises a data fitter 326 for fitting a temporal segment to a template segment by at least one of: scaling, applying a time shift, or applying an offset. For best results, these three parameters are fitted simultaneously.

Any kind of electrodes may be used, however a capacitive electrode for acquiring the composite signal may be more comfortable for the patient and more efficient to apply.

The system further provides the capabilities to display, store, and/or transmit the residual signal and/or the extracted ECG signal. For example, a display 328 is provided for showing at least one of the established signals; a recorder for storing at least one of the established signals; or an output for transmitting at least one of the established signals to another device.

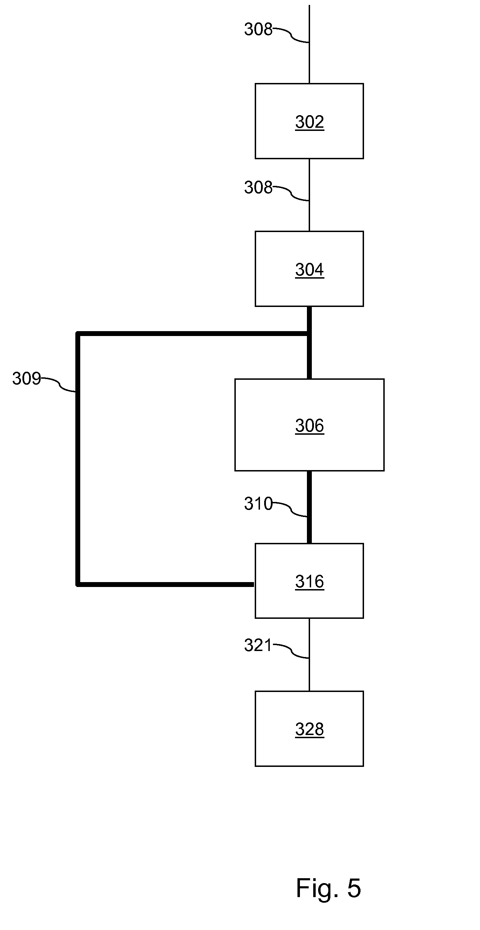

Fig. 5 illustrates an alternative embodiment, wherein the same numerals are used as in Fig. 4 to denote similar objects and signals. This embodiment comprises means 316 for subtracting the template segments 310 from the corresponding temporal segments 309 of the composite signal 308 to obtain the residual signal 321. This way, the complete (maternal) ECG signal does not need to be extracted. Instead, only the template segments need to be computed. In an embodiment, only template segements for the most prominent predetermined segments are computed, and the less prominent predetermined segments are not computed. For example, only the R wave segment is computed. In another example, the isoelectrical segments are omitted.

The systems described above may be implemented in a cardiac monitoring device.

An embodiment comprises a method of extracting an ECG signal from a composite signal, the method comprising the steps of

> receiving a composite signal 308 comprising an ECG signal;
> identifying a plurality of temporal segments 309 of the composite signal corresponding to a plurality of predetermined segments of an ECG complex 208; and
> generating a plurality of respective template segments 310 corresponding to the respective predetermined segments, wherein a respective template segment is based on a plurality of the corresponding identified temporal segments.

It will be appreciated that the invention also extends to computer programs, particularly computer programs on or in a carrier, adapted for putting the invention into practice. The program may be in the form of source code, object code, a code intermediate source and object code such as partially compiled form, or in any other form suitable for use in the implementation of the method according to the invention. It will also be appreciated that such a program may have many different architectural designs. For example, a program code implementing the functionality of the method or system according to the invention may be subdivided into one or more subroutines. Many different ways to distribute the functionality among these subroutines will be apparent to the skilled person. The subroutines may be stored together in one executable file to form a self-contained program. Such an executable file may comprise computer executable instructions, for example processor instructions and/or interpreter instructions (e.g. Java interpreter instructions). Alternatively, one or more or all of the subroutines may be stored in at least one external library file and linked with a main program either statically or dynamically, e.g. at run-time. The main program contains at least one call to at least one of the subroutines. Also, the subroutines may comprise function calls to each other. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the means of at least one of the systems and/or products set forth. These instructions may be subdivided into subroutines and/or be stored in one or more files that may be linked statically or dynamically.

The carrier of a computer program may be any entity or device capable of carrying the program. For example, the carrier may include a storage medium, such as a ROM, for example a CD ROM or a semiconductor ROM, or a magnetic recording medium, for example a floppy disc or hard disk. Further the carrier may be a transmissible carrier such as an electrical or optical signal, which may be conveyed via electrical or optical cable or by radio or other means. When the program is embodied in such a signal, the carrier may be constituted by such cable or other device or means. Alternatively, the carrier may be an integrated circuit in which the program is embedded, the integrated circuit being adapted for performing, or for use in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1.   A system for extracting an ECG signal from a composite signal, the system comprising

> an input (302) for receiving a composite signal (308) representing an electric measurement of a living subject;
> identification means (304) for identifying a plurality of temporal segments (309) of the composite signal corresponding to a plurality of predetermined segments (202, 204, 206) of an ECG complex (208); and
a template generator (306) for generating a plurality of respective template segments (310) corresponding to the respective predetermined segments (202, 204, 206), wherein a respective template segment is based on a plurality of the corresponding identified temporal segments, the template segments representing extracted portions of the ECG signal.

2. The system according to claim 1, further comprising a signal generator (312) for combining a plurality of the template segments (310) to obtain an extracted ECG signal (314).

3. The system according to claim 2, further comprising a subtractor (322) for subtracting the extracted ECG signal (314) from the composite signal (308) to obtain a residual signal (320).

4. The system according to any one of the preceding claims, wherein the template generator (306) comprises means (318) for updating a template segment to take into account temporal segments in a predetermined time interval around or near a temporal segment of interest to obtain a time-varying adaptive template segment.

5. The system according to any one of the preceding claims, further comprising means (316) for subtracting the template segments (310) from the corresponding temporal segments (309) of the composite signal (308) to obtain a residual signal (321).

6. The system according to any one of the preceding claims, wherein

   the input is arranged for receiving a maternal ECG signal and a fetal ECG signal mixed in the composite signal; and wherein
   the predetermined segments of the ECG complex are predetermined segments of a maternal ECG complex.

7. The system according to claim 6 in dependence on claim 5, further comprising

   a postprocessor for analyzing the residual signal, which comprises the fetal ECG signal; and
   means for generating an alarm upon detecting a predetermined event in the fetal ECG signal.

8. The system according to any one of claims 1 to 5, wherein

   the input is arranged for receiving the ECG signal and an EMG signal mixed in the composite signal.

9. The system according to any one of the preceding claims, wherein the predetermined segments of the ECG complex comprise at least part of at least one of: a P wave, a Q wave, an R wave, an S wave, a T wave.

10. The system according to claim 9, wherein the identification means comprises a means for finding an extreme value and an adaptive threshold to identify a boundary of at least one of the segments.

11. The system according to any one of the preceding claims,
    wherein the template generator (306) comprises an outlier detector (324) for detecting outliers among samples within a segment; and
    wherein the template generator is arranged for not taking into account the outliers in determining the template segments.

12. The system according to claim 11, further comprising

    a confidence estimator for providing a confidence measure of the composite signal based on the outliers to assess a relevance of an electrode used to acquire the composite signal in a multi-electrode configuration.

13. The system according to any one of the preceding claims, wherein the template generator (306) comprises a data fitter (326) for fitting a temporal segment to a template segment by at least one of: scaling, applying a time shift, or applying an offset.

14. The system according to claim 1, further comprising a capacitive electrode for acquiring the composite signal.

**15.** The system according to any one of the preceding claims, wherein the system further comprises at least one of:

a display (328) for showing at least one of the established signals;
a recorder for storing at least one of the established signals; or
an output for transmitting at least one of the established signals to another device.

**16.** A cardiac monitoring device comprising a system according to any one of the preceding claims.

**17.** A method of extracting an ECG signal from a composite signal, the method comprising

receiving a composite signal (308) representing an electric measurement of a living subject;
identifying a plurality of temporal segments (309) of the composite signal corresponding to a plurality of predetermined segments of an ECG complex (208); and
generating a plurality of respective template segments (310) corresponding to the respective predetermined segments, wherein a respective template segment is based on a plurality of the corresponding identified temporal segments, the template segments representing extracted portions of the ECG signal.

**18.** A computer program product comprising machine executable instructions for causing a processor to perform the method according to claim 17.

Fig. 1A

Fig. 1B

Fig. 2

Fig. 3

Fig. 4

308

302

308

304

309

306

310

316

321

328

Fig. 5

**European Patent Office**

## EUROPEAN SEARCH REPORT

Application Number

EP 07 11 2842

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RIK VULLINGS ET AL: "Maternal ECG removal from non-invasive fetal ECG recordings" ENGINEERING IN MEDICINE AND BIOLOGY SOCIETY, 2006. EMBS '06. 28TH ANNUAL INTERNATIONAL CONFERENCE OF THE IEEE, IEEE, PI, August 2006 (2006-08), pages 1394-1397, XP031029011 ISBN: 1-4244-0032-5 | 1-6, 8-10,13, 14,16-18 | INV. A61B5/0444 G06F17/00 |
| Y | * the whole document * | 7,15 | |
| D,X | UNGUREANU ET AL: "Basic Aspects Concerning the Event-Synchronous Interference Canceller" IEEE TRANSACTIONS ON BIOMEDICAL ENGINEERING, IEEE SERVICE CENTER, PISCATAWAY, NJ, US, vol. 53, no. 11, November 2006 (2006-11), pages 2240-2247, XP011149487 ISSN: 0018-9294 abstract, sections II and VI | 1-3,6,8, 9,13,17 | |
| X | M UNGUREANU ET AL: "Fetal ECG extraction during labor using an adaptive maternal beat subtraction technique" BIOMEDIZINISCHE TECHNIK, vol. 52, no. 1, February 2007 (2007-02), pages 56-60, XP001537747 * page 57, left-hand column, paragraph 2 - page 58, right-hand column, paragraph 4 * | 1-3,6,8, 9,11,13, 17 | TECHNICAL FIELDS SEARCHED (IPC) A61B G06F |
| Y | WO 2005/039410 A (UNIV NOTTINGHAM [GB]; CROWE JOHN ANDREW [GB]; JAMES DAVID [GB]; HAYES-) 6 May 2005 (2005-05-06) * page 12, lines 11-16 * * page 14, line 19 - page 16, line 6 * * page 17, line 28 - page 28, line 4; figure 8 * | 7,15 | |

-/--

The present search report has been drawn up for all claims

| Place of search | Date of completion of the search | Examiner |
|---|---|---|
| Munich | 18 December 2007 | Küster, Gunilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 07 11 2842

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 01/26545 A (UNIV NOTTINGHAM [GB]; HAYES GILL BARRIE [GB]; JAMES DAVID [GB]; CROWE) 19 April 2001 (2001-04-19) * page 23, line 26 - page 25, line 33 * ----- | 1,17 | |
| | | | **TECHNICAL FIELDS SEARCHED (IPC)** |

The present search report has been drawn up for all claims

| Place of search | Date of completion of the search | Examiner |
|---|---|---|
| Munich | 18 December 2007 | Küster, Gunilla |

EPO FORM 1503 03.82 (P04C01)

**ANNEX TO THE EUROPEAN SEARCH REPORT
ON EUROPEAN PATENT APPLICATION NO.**

EP 07 11 2842

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2007

| Patent document cited in search report | | Publication date | Patent family member(s) | | Publication date |
|---|---|---|---|---|---|
| WO 2005039410 | A | 06-05-2005 | EP | 1680018 A1 | 19-07-2006 |
| | | | US | 2007213627 A1 | 13-09-2007 |
| WO 0126545 | A | 19-04-2001 | AU | 7673400 A | 23-04-2001 |
| | | | EP | 1220640 A1 | 10-07-2002 |

EPO FORM P0459

For more details about this annex : see Official Journal of the European Patent Office, No. 12/82

**EP 2 016 894 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. UNGUREANU et al.** Basic aspects concerning the event-synchronous interference canceller. *IEEE Transactions on Biomedical Engineering,* November 2006, vol. 53 (11